# EUROPÄISCHE PATENTSCHRIFT

(11) **EP 1 753 715 B1**
(45) Veröffentlichungstag und Bekanntmachung des Hinweises auf die Patenterteilung: **07.08.2019**
(21) Anmeldenummer: 05752554.5
(22) Anmeldetag: 21.05.2005
(51) Int. Cl.: C07C 263/10, C07C 265/12, C07C 265/14

(54) **VERFAHREN ZUR HERSTELLUNG VON ISOCYANATEN**
ISOCYANATE PRODUCTION METHOD
PROCEDE DE PRODUCTION D'ISOCYANATES

(30) Priorität: 25.05.2004 DE 102004026095
(43) Veröffentlichungstag der Anmeldung: 21.02.2007
(73) Patentinhaber: BASF SE, 67056 Ludwigshafen am Rhein (DE)
(72) Erfinder: WÖLFERT, Andreas, 74906 Bad Rappenau (DE); PALLASCH, Hans-Jürgen, 67063 Ludwigshafen (DE); STROEFER, Eckhard, 68163 Mannheim (DE); PENZEL, Ulrich, 01945 Tettau (DE); DEBERDT, Filip, B-2812 Muizen (BE)
(74) Vertreter: BASF IP Association
(86) Internationale Anmeldenummer: PCT/EP2005/005520
(87) Internationale Veröffentlichungsnummer: WO 2005/115974

(56) Entgegenhaltungen:
- EP-A- 0 150 435
- WO-A-96/16028
- WO-A-2004/058689
- DE-A1- 1 768 439

## Beschreibung

Verfahren zur Herstellung von Isocyanaten durch Umsetzung von Aminen mit Phosgen, wobei der Phosgenstrom, welcher der Vermischung von Amin- und Phosgen zugeführt wird,
(i) im Wesentlichen frei von Isocyanaten ist und
(ii) einen Massengehalt an Chlorwasserstoff von weniger als 0,4 Massen-% aufweist, wobei die benötigte Menge an HCl im phosgenhaltigen Eduktstrom (11) durch Zusatz von HCl im frischen Phosgen (1) bereitgestellt wird oder im rückgeführten Phosgenstrom (10) durch Aufarbeitung in der Phosgenaufarbeitung (IX) unter Berücksichtigung der zugeführten Menge frischen Phosgens (1) eingestellt wird.

Es sind in der Literatur bereits verschiedene Verfahren zur Herstellung von Isocyanaten durch Umsetzung von Aminen mit Phosgen beschrieben.

EP-A-322 647 beschreibt die kontinuierliche Herstellung von Mono- oder Polyisocyanaten in einer Kombination aus einer Mischdüse, in der der Aminstrom und der Phosgenstrom intensiv vermischt werden und einem nachgeschalteten senkrechten Rohrreaktor, der durch Lochböden kaskadiert wird. Nachteilig an diesem Verfahren ist die Verstopfungsneigung in der Mischdüse, was die Betriebsstabilität des Verfahrens reduziert.

So beschreibt deshalb EP 0830 894 die Verwendung von Reinigungsstiften in der Mischeinrichtung, um die Betreibbarkeit der Mischdüse zu verbessern. Nachteilig ist die Verwendung von bewegten Teilen in der Mischeinrichtung. An den Durchführungsstellen besteht das Risiko eines unerwünschten Austritts des giftigen Phosgens. Weitere Ansätze hinsichtlich der Optimierung des Verfahrens bestehen in der Optimierung der eingesetzten Eduktströme, um das Verfahren hinsichtlich der RaumZeitausbeute zu verbessern.

WO 96/16028 beschreibt ein kontinuierliches Verfahren zur Herstellung von Isocyanaten, gekennzeichnet durch einstufige Reaktionsführung hinsichtlich der Temperatur, Verwendung von Isocyanat als Lösungsmittel für das Phosgen, wobei der Chlorgehalt des Isocyanats kleiner als 2 % ist. Für die Phosgenierung kann ein Rohrreaktor eingesetzt werden. Nachteilig an dem Verfahren ist, dass das Isocyanat kontinuierlich in die Reaktionszone zurückgeführt wird, wo es in Anwesenheit des freien Amins zu Harnstoffen reagieren kann, die als Feststoff ausfallen. Der stabile Betrieb eines derartigen Verfahrens ist aber durch die Feststoffproblematik gefährdet. Durch die hohe im Kreis geführte Menge Isocyanat ergibt sich ein relativ großes Reaktionsvolumen, was mit einem unerwünscht hohen apparativen Aufwand verbunden ist.

US 4,581,174 beschreibt die kontinuierliche Herstellung von organischen Mono- und/oder Polyisocyanaten durch Phosgenierung des primären Amines in einem Mischkreis unter teilweiser Rückführung der isocyanathaltigen Reaktionsmixtur, wobei der HCl-Anteil in der rückgeführten Mischung kleiner als 0,5 % ist. Auch hier gilt, dass die kontinuierliche Rückführung des Isocyanates in die Reaktionszone mit freiem Amin die Harnstoffbildung fördert. Der ausfallende Harnstoff gefährdet den stabilen Betrieb des Verfahrens.

GB 737 442 beschreibt die Rückgewinnung von Phosgen aus der Isocyanatsynthese. Das rückgewonnene Phosgen hat einen HCl-Gehalt von 0,5 bis 0,7 %.

EP 0 150 435 offenbart ebenfalls ein Verfahren zur Herstellung von Isocyanaten durch Umsetzung von Aminen mit Phosgen.

Es ist weiterhin bekannt, dass die Verwendung eines hohen Phosgenüberschusses gegenüber dem verwendeten Amin zu hohen Selektivitäten bezüglich des hergestellten Isocyanates führt und somit einen entscheidenden Einfluss auf die Wirtschaftlichkeit des Herstellungsverfahrens hat. Mit zunehmendem Verhältnis von Phosgen zu Aminogruppen steigt tendenziell der Phosgen-Hold-up der Anlage und das Anlagenvolumen. Andererseits wird aufgrund der Giftigkeit von Phosgen ein möglichst geringer Phosgen-Hold-Up und ein möglichst kompakter Bau der Anlage angestrebt. Dies stellt gleichzeitig eine Reduzierung der Investitionskosten der Anlage und somit eine Verbesserung der Wirtschaftlichkeit des Verfahrens dar.

Ein weiterer Aspekt für das Erreichen einer guten Wirtschaftlichkeit ist eine hohe Anlagenlaufzeit ohne Abstellung der Anlage. Abstellungen sind bei den Isocyanatproduktionsanlagen üblicherweise dann notwendig, wenn Anlagenteile durch die während der Phosgenierung entstehenden Feststoffe verstopfen.

Aufgabe der Erfindung war es daher, ein Verfahren zur Herstellung von Isocyanaten bereitzustellen, welches es gestattet, dass die resultierenden Reaktionen unter hoher Selektivität und hoher Raum-Zeit-Ausbeute und hoher Betriebsstabilität durchgeführt werden, so dass das Verfahren räumlich kompakt aufgebaut werden kann und wirtschaftlich betrieben werden kann.

Insbesondere war es Aufgabe der Erfindung, ein Verfahren zur Herstellung von Isocyanaten bereit zu stellen, welches eine niedrige Feststoffbildung und Feststoffablagerung in der Produktionsanlage, insbesondere in der Mischdüse und im Verweilzeitreaktor, ermöglicht.

Es wurde nun gefunden, dass das Verfahren hinsichtlich seiner Betreibbarkeit dann besonders effektiv betrieben werden kann, wenn zum einen auf die im Stand der Technik häufig beschriebene Rückführung von Isocyanaten oder die Verwendung von Isocyanaten als Lösungsmittel verzichtet wird und zum anderen der HCl-Gehalt in dem der Mischeinrichtung oder Reaktionseinrichtung zugeführten phosgenhaltigen Strom gering gehalten wird.

Gegenstand der Erfindung ist daher ein Verfahren zur Herstellung von Isocyanaten durch Umsetzung von Aminen mit Phosgen, wobei der Phosgenstrom, welcher der Vermischung von Amin- und Phosgen zugeführt wird,
(i) im Wesentlichen frei von Isocyanaten ist und
(ii) einen Massengehalt an Chlorwasserstoff von weniger als 0,4 Massen-% aufweist, wobei die benötigte Menge an HCl im phosgenhaltigen Eduktstrom (11) durch Zusatz von HCl im frischen Phosgen (1) bereitgestellt wird oder im rückgeführten Phosgenstrom (10) durch Aufarbeitung in der Phosgenaufarbeitung (IX) unter Berücksichtigung der zugeführten Menge frischen Phosgens (1) eingestellt wird.

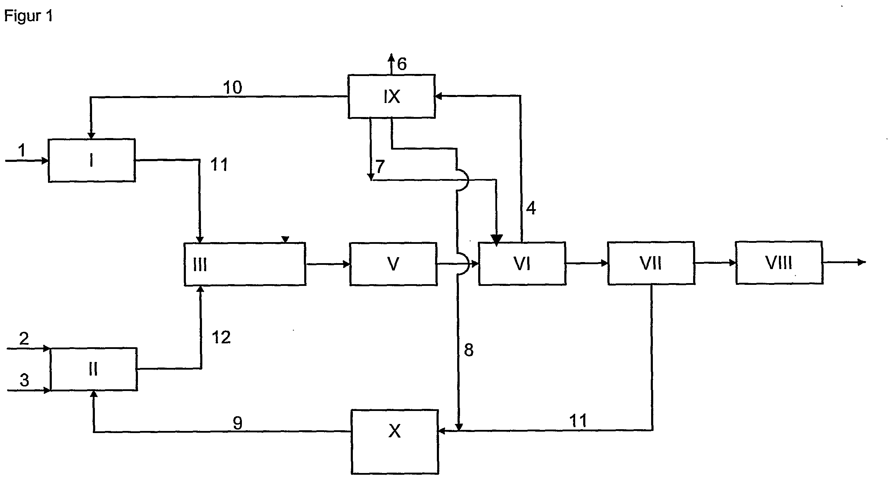

Eine bevorzugte Ausführungsform des erfindungsgemäßen Verfahrens ist in Figur 1 veranschaulicht. In Figur 1 bedeutet:

| | |
|---|---|
| I | Phosgenvorlage |
| II | Aminvorlage |
| III | Mischvorrichtung |
| V | Reaktor |
| VI | Erste Aufbereitungsvorrichtung |
| VII | Zweite Aufbereitungsvorrichtung (optional) |
| VIII | Isocyanatvorlage |
| IX | Phosgenaufarbeitung |
| X | Lösungsmittelaufarbeitung (optional) |
| 1 | Zufuhr frisches Phosgen |
| 2 | Zufuhr frisches Amin |
| 3 | Zufuhr inertes Lösungsmittel (optional) |
| 4 | Abgetrennter Chlorwasserstoff, Phosgen, inertes Lösungsmittel, und gegebenenfalls geringe Mengen Isocyanat |
| 6 | Ausgetragener Chlorwasserstoff |
| 7 | Abgetrenntes Isocyanat (optional) |
| 8, 11 | Abgetrenntes inertes Lösungsmittel (optional) |
| 9 | Aufgearbeitetes inertes Lösungsmittel (optional) |
| 10 | Aufgearbeitetes Phosgen |
| 11 | Phosgenhaltiger Eduktstrom mit den erfindungsgemäßen Merkmalen (i) und (ii) |
| 12 | Aminhaltiger Eduktstrom |

Das Amin aus der Aminvorlage II und das Phosgen, aus der Phosgenvorlage I werden in einer geeigneten Mischvorrichtung III vermischt. Die Phosgenvorlage I kann mit frischem Phosgen 1 oder mit rückgeführten und aufgearbeiteten Phosgen 10 befüllt werden. Der aus der Phosgenvorlage I in die Mischvorrichtung III überführte Stoffstrom ist der phosgenhaltige Eduktstrom 11 mit den erfindungsgemäßen Merkmalen (i) und (ii) Nach dem Vermischen wird das Gemisch in einen Reaktor V überführt. Ebenfalls sind Vorrichtungen verwendbar, die sowohl Misch- als auch Reaktionsvorrichtung darstellen (d.h. III und V wird in einer Vorrichtung kombiniert), beispielsweise Rohrreaktoren mit angeflanschten Düsen.

In der Aufbereitungsvorrichtung VI wird üblicherweise vom Isocyanatstrom Chlorwasserstoff und gegebenenfalls inertes Lösungsmittel und/oder geringe Anteile des Isocyanatstroms abgetrennt. In der optionalen Aufbereitungsvorrichtung VII wird bevorzugt inertes Lösungsmittel abgetrennt, anschließend in einer geeigneten Vorrichtung X aufgearbeitet und der Aminvorlage II wieder zugeführt. Beispielsweise können übliche Destillationseinheiten die Aufbereitungsvorrichtungen darstellen.

Es ist erfindungswesentlich, dass das zur Umsetzung benötigte und zugeführte Phosgen (=zugeführter phosgenhaltiger Eduktstrom 11) einen Massengehalt an Chlorwasserstoff von weniger als 0,4 Massen-% aufweist (ii). Bevorzugt weist der phosgenhaltige Eduktstrom einen Massengehalt an Chlorwasserstoff von 0,00001 % bis weniger als 0,4 Massen-%, mehr bevorzugt von 0,0001 % bis weniger als 0,3-Massen-%, besonders bevorzugt von 0,0005 % bis weniger als 0,25 Massen-% und ganz besonders bevorzugt von 0,001 % bis weniger als 0,2 Massen-% auf.

Im Rahmen dieser Erfindung bezieht sich die Angabe in Massenprozenten des phosgenhaltigen Eduktstroms (Merkmale (i) und (ii)) auf die Gesamtmasse der Summe aus Phosgen, HCl und gegebenenfalls Verunreinigungen an Isocyanat. Diese Angabe in Massenprozenten bezieht sich nicht auf die Masse des phosgenhaltigen Eduktstroms inklusive Lösungsmittel, falls der phosgenhaltige Eduktstrom, der zur Reaktions- oder Mischeinrichtung geführt wird, noch zusätzlich eines oder mehrere Lösungsmittel enthält.

Die benötigte Menge an HCl im phosgenhaltigen Eduktstrom 11 kann durch Zusatz von HCl im frischen Phosgen oder bevorzugt durch entsprechende Aufarbeitung des Phosgenstroms 10 (d.h. die Phosgenaufarbeitung IX wird so eingestellt, dass der Phosgenstrom 10 - unter Berücksichtigung der zugeführten Menge frischen Phosgens 1 - die erfindungsgemäße Menge an HCl (ii) im Strom 11 bereitstellt) erfolgen.

Ferner ist es erfindungswesentlich, dass der phosgenhaltige Eduktstrom im wesentlichen keine Isocyanate enthält (i). Darunter ist zu verstehen,
dass im erfindungsgemäßen Verfahren keine im Reaktor hergestellten Isocyanate (oder auch andere Isocyanatverbindungen) rückgeführt und dem phosgenhaltigen Eduktstrom zugeführt werden,
oder
dass Isocyanate als Lösungsmittel verwendet werden und dem phosgenhaltigen Eduktstrom zugeführt werden.

In der Aufarbeitungsvorrichtung VI wird HCl, Phosgen und gegebenenfalls inertes Lösungsmitte und auch geringe Mengen an Isocyanat vom Hauptproduktstrom (das herzustellende Isocyanat) abgetrennt. Die Abtrennung von Isocyanat in der Vorrichtung VI ist üblicherweise technisch bedingt, aber nicht erwünscht. Ferner wird im allgemeinen in der Phosgenaufarbeitung IX nochmals Isocyanat abgetrennt 7, dieses kann beispielsweise der ersten Aufarbeitungsvorrichtung VI zugeführt werden. Es kann jedoch technisch bedingt sein, dass die Aufarbeitungsvorrichtung VI keine vollständige, d.h. 100 %ige Isocyanatabtrennung leisten kann.

Im Rahmen dieser Erfindung ist es wichtig, dass der phosgenhaltige Eduktstrom im wesentlichen keine Isocyanate enthält (i). Dennoch kann wie vorstehend erläutert der phosgenhaltige Eduktstrom Isocyanate in geringen Mengen enthalten, beispielsweise, weil es technische nicht möglich ist, den Strom 10 vollständig rein von Isocyanaten zu bekommen. "Im wesentlichen keine Isocyanate" ist daher so zu verstehen, dass der phosgenhaltige Eduktstrom üblicherweise weniger als 1 Massen-%, bevorzugt von 0,00001 % bis weniger als 1 Massen-%, mehr bevorzugt von 0,0001 % bis weniger als 0,5-Massen-%, noch mehr bevorzugt von 0,001 % bis weniger als 0,3 Massen-%, besonders bevorzugt von 0,01 % bis weniger als 0,2 Massen-% an Isocyanaten enthält.

Unter "Isocyanate" werde im Rahmen dieser Erfindungen alle Verbindungen verstanden, die mindestens eine freie Isocyanatgruppe aufweisen.

Zur Berechnung der Menge an HCl und Isocyanaten im phosgenhaltigen Eduktstrom werden gegebenenfalls auch Carbamoylchloride herangezogen. Carbamoylchlorid bildet sich durch Umsetzung von Isocyanaten mit HCl in einer Gleichgewichtsreaktion. Sofern der phosgenhaltige Eduktstrom Carbamoylchloride enthält werden diese "theoretisch" in HCl und Phosgen gespalten und die jeweilige Menge der Spaltprodukte zur Berechnung der Mengen an Isocyanat (i) und HCl (ii) mit herangezogen.

Es ist weiterhin bevorzugt, dass der Phosgenstrom, welcher der Vermischung von Amin- und Phosgenstrom zugeführt wird, bereits die oben angegebene Menge an HCl enthält. Die Menge an HCl sollte nicht erst, wie in US 3,234,253 dargestellt, nachträglich im Reaktionsgemisch von Amin und Phosgen eingeführt werden.

lm erfindungsgemäßen Verfahren erfolgt die Vermischung der Reaktanten in einer Mischeinrichtung, die sich durch eine hohe Scherung des durch die Mischeinrichtung geführten Reaktionsstromes auszeichnet. Bevorzugt werden als Mischeinrichtung eine Rotationsmischeinrichtung, eine Mischpumpe oder eine Mischdüse verwendet, die dem Reaktor vorangestellt ist. Besonders bevorzugt wird eine Mischdüse verwendet. Die Mischzeit in dieser Mischeinrichtung beträgt üblicherweise 0,0001 s bis 5 s, bevorzugt 0,0005 bis 4 s, besonders bevorzugt 0,001 s bis 3 s. Als Mischzeit ist diejenige Zeit zu verstehen, die von dem Beginn des Mischvorgangs vergeht, bis 97,5 % der Fluidelemente des erhaltenen Gemisches einen Mischungsbruch haben, der bezogen auf den Wert des theoretischen Endwerts des Mischungsbruchs des erhaltenen Gemisches beim Erreichen des Zustandes perfekter Mischung weniger als 2,5 % von diesem Endwert des Mischungbruches abweichen. (zum Konzept des Mischungsbruches siehe z.B. J. Warnatz, U. Maas, R.W. Dibble: Verbrennung, Springer Verlag, Berlin Heidelberg New York, 1997, 2. Auflage, S. 134).

In einer bevorzugten Ausführungsform erfolgt die Umsetzung von Amin mit Phosgen bei Absolutdrücken von 0,9 bar bis 400 bar, bevorzugt von 3 bis 35 bar. Das molare Verhältnis von Phosgen zu eingesetzten Aminogruppen beträgt im allgemeinen 1,1 : 1 bis 12 : 1, bevorzugt von 1,25:1 bis 8:1. Die Gesamtverweilzeit in den Reaktoren beträgt in allgemeinen 10 Sekunden bis 15 Stunden, bevorzugt 3 min bis 12 h. Die Umsetzungstemperatur beträgt im allgemeinen von 25 bis 260°C (Grad Celsius), bevorzugt von 35 bis 240°C.

Das erfindungsgemäße Verfahren eignet sich für die Herstellung alle gängigen aliphatischen und aromatischen Isocyanate, oder ein Gemisch aus zwei oder mehr solcher Isocyanate. Bevorzugt werden beispielsweise monomeres Methylen-di(phenylisocyanat) (m-MDI) oder polymeres Methylen-di(phenylisocyanat (p-MDI), Toluylendiisocyanat (TDI), R,S-1-Phenylethylisocyanat, 1-Methyl-3-phenylpropylisocyanat, Naphthyldiisocyanat (NDI), n-Pentylisocyanat, 6-Methyl-2-heptanisocyanat, Cyclopentylisocyanat, Hexamethylendiisocyanat (HDI), Isophorondiisocyanat (IPDI), Di-isocyanato-methyl-cyclohexan (H₆TDI), Xylendiisocyanat (XDI), Di-isocyanato-cyclohexan (t-CHDI), Di-(isocyanato-cyclohexyl)-methan (H₁₂MDI).

Besonders bevorzugt wird das Verfahren zur Herstellung von TDI, m-MDI, p-MDI, HDI, IPDI, H₆TDI, H12MDI, XDI, t-CHDI und NDI, insbesondere zur Herstellung von TDI eingesetzt.

Das erfindungsgemäße Verfahren umfasst kontinuierliche, halbkontinuierliche und diskontinuierliche Verfahren. Bevorzugt sind kontinuierliche Verfahren.

Die Herstellung der Isocyanate erfolgt üblicherweise durch Umsetzung des entsprechenden primären Amines mit einem Überschuss an Phosgen. Dabei findet dieser Prozess bevorzugt in der flüssigen Phase statt.

Dem erfindungsgemäßen Verfahren kann ein zusätzliches inertes Lösungsmittel beigesetzt werden. Dieses zusätzliche inerte Lösungsmittel ist üblicherweise ein organisches Lösungsmittel oder Gemische davon. Dabei sind Chlorbenzol, Dichlorbenzol, Trichlorbenzol, Toluol, Hexan, Diethylisophthalat (DEIP), Tetrahydrofuran (THF), Dimethylformamid (DMF), Benzol und deren Gemische bevorzugt. Das Isocyanat, das in der Anlage hergestellt, kann entgegen zahlreicher Publikationen im Stand der Technik nicht als Lösungsmittel verwendet werden. Besonders bevorzugt ist als Lösungsmittel Chlorbenzol.

Der Gehalt an Amin bezogen auf das Gemisch Amin/Lösungsmittel beträgt üblicherweise zwischen 1 und 50 Massen-%, bevorzugt zwischen 2 und 40 Massen-%, besonders bevorzugt zwischen 3 und 30 Massen-%.

Nach der Reaktion wird das Stoffgemisch bevorzugt mittels Rektifikation in Isocyanat(e), Lösungsmittel, Phosgen und Chlorwasserstoff aufgetrennt. Geringe Mengen von Nebenprodukten, die im Isocyanat(e) verbleiben, können mittels zusätzlicher Rektifikaktion oder auch Kristallisation vom erwünschten Isocyanat(e) getrennt werden.

Je nach Wahl der Reaktionsbedingungen kann das Produkt inertes Lösungsmittel, Carbamoylchlorid und/oder Phosgen enthalten und nach den bekannten Methoden weiterverarbeitet werden.

Bei der Umsetzung reagiert das Phosgen zunächst mit den Aminogruppen zum sogenannten Carbamoylchlorid unter Abspaltung von Chlorwasserstoff. Die Carbamoylchloridgruppe setzt sich dann unter weiterer Abspaltung von Chlorwasserstoff zur Isocyanatgruppe um.

Nach beendeter Reaktion werden üblicherweise der gebildete Chlorwasserstoff und das überschüssige Phosgen aus der Reaktionsmischung durch Destillation oder durch Strippung mit einem Inertgas abgetrennt. Das Chlorwasserstoff/Phosgengemisch wird üblicherweise durch Destillation (FR 1 469 105) oder durch Wäsche mit einem Kohlenwasserstoff in Chlorwasserstoff und Phosgen getrennt, wobei der Aufwand für die Trennung von HCl und Phosgen durch die Reinheitsanforderungen an die HCl bzw and das Phosgen bestimmt wird. Hierbei ist der Gehalt an Phosgen im HCl und der Gehalt an HCl im Phosgen zu unterscheiden. Das so erhaltene, vom HCl befreite Phosgen wird mit frischem Phosgen aus der Phosgensynthese vermischt und wieder der Reaktion zur Herstellung des Isocyanates zugeführt.

Je nach Betriebsweise der Anlage enthält der phosgenhaltige Strom, der der Reaktions- oder Mischeinrichtung zugeführt wird, neben Phosgen und den erwähnten Anteilen HCl auch noch das Lösungsmittel, in dem die Phosgenierung durchgeführt wird. Dies ist insbesondere dann der Fall, wenn die Trennung des Phosgens und des Chlorwasserstoffs mittels Wäsche mit dem Lösungsmittel durchgeführt wird.

Die zur Durchführung des erfindungsgemäßen Verfahrens geeignete Produktionsanlage umfasst die Vorrichtungen I, II, III, V, VI und gegebenenfalls die Vorrichtungen VII, VIII, IX und IX gemäß Figur 1. Es ist wesentlich, dass das in der Phosgenvorlage I befindliche Phosgen
(i) einen Massengehalt an Isocyanat von 0,00001 bis 1 %, bevorzugt von 0,0001 % bis weniger als 0,5-Massen-%, mehr bevorzugt von 0,001 % bis weniger als 0,3 Massen-%, besonders bevorzugt von 0,01 % bis weniger als 0,2 Massen-%, und
(ii) einen Massengehalt an Chlorwasserstoff von 0,00001 % bis weniger als 0,4 Massen-%, bevorzugt von 0,0001 % bis weniger als 0,3-Massen-%, besonders bevorzugt von 0,0005 % bis weniger als 0,25 Massen-% und ganz besonders bevorzugt von 0,001 % bis weniger als 0,2 Massen-% aufweist.

Das erfindungsgemäße Verfahren bringt somit den Vorteil mit sich, dass die Anzahl der Abstellungen zur Reinigung der Düse und damit die Anzahl der Anlagenstillstände im Vergleich zu bekannten Verfahren erheblich reduziert werden können. Zur Reinigung der Düse muss üblicherweise die Anlage phosgenfrei gemacht werden und dann geöffnet werden. Gleichzeitig wurde durch die Reduzierung der Öffnungen also auch die Bedienbarkeit der Anlage verbessert. Der technische Effekt des erfindungsgemäßen Verfahrens ist insbesondere deshalb überraschend, weil während der Reaktion der Isocyanatbildung ein Vielfaches des durch die Rückführung des überschüssigen Phosgens in das Verfahren eingetragenen Chlorwasserstoffs gebildet wird.

### Erfindungsgemäßes Beispiel 1

In der koaxialen Doppelrohrmischdüse einer Versuchsanlage wurden ein Toluylen-Lösungsstrom und ein Phosgen-Lösungsstrom vermischt. Der Toluylendiamin-Lösungsstrom von 5 kg/h, der zu 85 Gewichts-% aus Monochlorbenzol (MCB) und zu 15 Gewichts-% aus Toluylendiamin (TDA) bestand, wobei das TDA zu 80 Gewichts-% aus 2,4-TDA und zu 20 Gewichts-% aus 2,6-TDA bestand, wurde mit einer Geschwindigkeit von 7 m/s über das innere Rohr eingedüst. Der Phosgen-Lösungsstrom von 6,78 kg/h wurde über den äußeren Ringspalt mit einer Geschwindigkeit von 5,8 m/s zugeführt. Der Phosgen-Lösungsstrom enthielt neben 90 Gewichts-% Phosgen und mindestens 9 Gewichts-% MCB 0,02 Gewichts-% Chlorwasserstoff (HCl). Die Temperaturen der Eduktströme wurden so eingestellt, dass der aus der Mischdüse austretende Strom eine Temperatur von 150°C hatte. Dieser Strom wurde dann durch einen Rührkessel mit einer Verweilzeit von 20 min geleitet. Der Druck im Rührkessel betrug 10 bar abs. Der flüssige Reaktionsaustrag wurde über ein Standregelventil, der gasförmige Reaktionsaustrag wurde über ein Druckhalteventil auf eine Kolonne geleitet, in der ein Phosgen- und Chlorwasserstoffhaltiger Strom abdestilliert wurden. Die Kolonne arbeitete auf einem Druck von 3,5 bar abs.. Die Anlage wurde 72 Stunden ohne Verstopfungsprobleme betrieben.

### Vergleichsbeispiel 2

Wie in Beispiel 1 wurden in der koaxialen Doppelrohrmischdüse einer Versuchsanlage ein Toluylendiamin-Lösungsstrom und ein Phosgen-Lösungsstrom vermischt. Der Toluylendiamin-Lösungsstrom von 5 kg/h, der zu 85 Gewichts-% aus Monochlorbenzol (MCB) und zu 15 Gewichts-% aus Toluylendiamin (TDA) bestand, wobei das TDA zu 80 Gewichts-% aus 2,4-TDA und zu 20 Gewichts-% aus 2,6-TDA bestand, wurde mit einer Geschwindigkeit von 7 m/s über das innere Rohr eingedüst. Der Phosgen-Lösungsstrom von 6,78 kg/h wurde über den äußeren Ringspalt mit einer Geschwindigkeit von 5,8 m/s zugeführt. Der Phosgen-Lösungsstrom enthielt neben 90 Gewichts-% Phosgen und mindestens 9 Gewichts-% MCB noch 0,70 Gewichts-% Chlorwasserstoff (HCl). Die Temperaturen der Eduktströme wurden so eingestellt, dass der aus der Mischdüse austretende Strom eine Temperatur von 150°C hatte. Dieser Strom wurde dann durch einen Rührkessel mit einer Verweilzeit von 20 min geleitet. Der Druck im Rührkessel betrug 10 bar abs. Der flüssige Reaktionsaustrag wurde über ein Standregelventil, der gasförmige Reaktionsaustrag wurde über ein Druckhalteventil auf eine Kolonne geleitet, in der ein Phosgen- und Chlorwasserstoffhaltiger Strom abdestilliert wurden. Die Kolonne arbeitete auf einem Druck von 3,5 bar abs.. Nach 3,6 h musste die Anlage wegen Verstopfungsproblemen im Standregelventil des Rührkessels abgeschalten werden.

## Patentansprüche

1. Verfahren zur Herstellung von Isocyanaten durch Umsetzung von Aminen mit Phosgen, wobei der Phosgenstrom, welcher der Vermischung von Amin- und Phosgen zugeführt wird,
(i) im Wesentlichen frei von Isocyanaten ist und
(ii) einen Massengehalt an Chlorwasserstoff von weniger als 0,4 Massen-% aufweist, wobei die benötigte Menge an HCl im phosgenhaltigen Eduktstrom (11) durch Zusatz von HCl im frischen Phosgen (1) bereitgestellt wird oder im rückgeführten Phosgenstrom (10) durch Aufarbeitung in der Phosgenaufarbeitung (IX) unter Berücksichtigung der zugeführten Menge frischen Phosgens (1) eingestellt wird.

2. Verfahren nach Anspruch 1, **dadurch gekennzeichnet, dass** das Verfahren in einer Produktionsanlage aufgebaut aus einer Aminvorlage, einer Phosgenvorlage, einer Mischvorrichtung, einem Reaktor und einer Aufarbeitungsvorrichtung durchgeführt wird, wobei das in der Phosgenvorlage befindliche Phosgen
(i) einen Massengehalt an Isocyanat von 0,00001 bis 1 % und
(ii) einen Massengehalt an Chlorwasserstoff von 0,00001 % bis weniger als 0,4 Massen-% aufweist.

3. Verfahren nach Anspruch 1 oder 2, **dadurch gekennzeichnet, dass** der Phosgenstrom, welcher der Vermischung von Amin- und Phosgen zugeführt wird, einen Massengehalt an Chlorwasserstoff von 0,00002 % bis 0,3 % aufweist.

4. Verfahren nach einem der Ansprüche 1 bis 3, **dadurch gekennzeichnet, dass** der Phosgenstrom, welcher der Vermischung von Amin- und Phosgen zugeführt wird, mit einem Aminstrom, welcher der Vermischung von Amin- und Phosgen zugeführt wird, in einer Mischzeit von 0,0001 Sekunden bis 5 Sekunden vermischt wird.

5. Verfahren nach einem der Ansprüche 1 bis 3, **dadurch gekennzeichnet, dass** es zur Herstellung von TDI, m-MDI, p-MDI, HDI, IPDI, H6TDI, H12MDI, XDI, t-CHDI und NDI eingesetzt wird.

6. Verfahren nach einem der Ansprüche 1 bis 4, **dadurch gekennzeichnet, dass** die Umsetzung in einem Temperaturbereich von 25 bis 260°C und bei Absolutdrücken von 0,9 bar bis 400 bar durchgeführt wird, wobei das molare Verhältnis von Phosgen zu eingesetzten Aminogruppen 1,1 : 1 bis 12 : 1 beträgt.

7. Verfahren nach einem der Ansprüche 1 bis 6 **dadurch gekennzeichnet, dass** es in einem kontinuierlichen Verfahren durchgeführt wird und die Umsetzung von Phosgen mit Amin in der Flüssigphase erfolgt.

8. Verfahren nach einem der Ansprüche 1 bis 7 **dadurch gekennzeichnet, dass** das molare Verhältnis von Phosgen zu Aminogruppen 1,1 : 1 bis 12 : 1 beträgt.

## Claims

1. A process for the preparation of isocyanates by reacting amines with phosgene, wherein the phosgene stream which is fed to the mixing of amine and phosgene
(i) is substantially free of isocyanates and
(ii) has a mass content of hydrogen chloride of less than 0.4% by mass, wherein the required amount of HCl in the phosgene-containing stream (11) of starting materials is provided by addition of HCl to the fresh phosgene (1) or is established in the recycled phosgene stream (10) by workup in the phosgene workup (IX), taking account of the amount of fresh phosgene (1) supplied.

2. The process according to claim 1, which is carried out in a production plant comprising an amine vessel, a phosgene vessel, a mixing apparatus, a reactor and a working-up apparatus, wherein the phosgene present in the phosgene vessel
(i) has a mass content of isocyanate of from 0.00001 to 1% and
(ii)a mass content of hydrogen chloride of from 0.00001% to less than 0.4% by mass.

3. The process according to claim 1 or 2, wherein the phosgene stream which is fed to the mixing of amine and phosgene has a mass content of hydrogen chloride of from 0.00002 % to 0.3%.

4. The process according to any of claims 1 to 3, wherein the phosgene stream which is fed to the mixing of amine and phosgene is mixed with an amine stream which is fed to the mixing of amine and phosgene in a mixing time of from 0.0001 second to 5 seconds.

5. The process according to any of claims 1 to 3, which is used for the preparation of TDI, m-MDI, p-MDI, HDI, IPDI, H6TDI, H12MDI, XDI, t-CHDI and NDI.

6. The process according to any of claims 1 to 4, wherein the reaction is carried out in a temperature range from 25 to 260°C and at absolute pressures of from 0.9 bar to 400 bar, the molar ratio of phosgene to amino groups used being from 1.1 : 1 to 12 : 1.

7. The process according to any of claims 1 to 6, wherein said process is carried out by a continuous method and the reaction of phosgene with amine is effected in the liquid phase.

8. The process according to any of claims 1 to 7, wherein the molar ratio of phosgene to amino groups is from 1.1 : 1 to 12 : 1.

## Revendications

1. Procédé de fabrication d'isocyanates par mise en réaction d'amines avec du phosgène, le courant de phosgène qui est introduit dans le mélange d'amine et de phosgène,
(i) étant essentiellement exempt d'isocyanates et
(ii) présentant une teneur massique en chlorure d'hydrogène de moins de 0,4 % en masse, la quantité nécessaire d'HCl dans le courant de réactifs contenant du phosgène (11) étant mise à disposition par ajout d'HCl dans le phosgène frais (1) ou étant ajustée dans le courant de phosgène recyclé (10) par traitement lors du traitement du phosgène (IX) en prenant en compte la quantité introduite de phosgène frais (1).

2. Procédé selon la revendication 1, **caractérisé en ce que** le procédé est réalisé dans une installation de production constituée par un réservoir d'amine, un réservoir de phosgène, un dispositif de mélange, un réacteur et un dispositif de traitement, le phosgène qui se trouve dans le réservoir de phosgène présentant :
(i) une teneur massique en isocyanate de 0,00001 à 1 % et
(ii) une teneur massique en chlorure d'hydrogène de 0,00001 % à moins de 0,4 % en masse.

3. Procédé selon la revendication 1 ou 2, **caractérisé en ce que** le courant de phosgène qui est introduit dans le mélange d'amine et de phosgène présente une teneur massique en chlorure d'hydrogène de 0,00002 % à 0,3 %.

4. Procédé selon l'une quelconque des revendications 1 à 3, **caractérisé en ce que** le courant de phosgène, qui est introduit dans le mélange d'amine et de phosgène, est mélangé avec un courant d'amine, qui est introduit dans le mélange d'amine et de phosgène, en un temps de mélange de 0,0001 seconde à 5 secondes.

5. Procédé selon l'une quelconque des revendications 1 à 3, **caractérisé en ce qu'**il est utilisé pour la fabrication de TDI, de m-MDI, de p-MDI, d'HDI, d'IPDI, d'H6TDI, d'H12MDI, de XDI, de t-CHDI et de NDI.

6. Procédé selon l'une quelconque des revendications 1 à 4, **caractérisé en ce que** la réaction est réalisée dans une plage de température allant de 25 à 260°C et à des pressions absolues de 0,9 bar à 400 bar, le rapport molaire entre le phosgène et les groupes amino utilisés étant de 1,1:1 à 12:1.

7. Procédé selon l'une quelconque des revendications 1 à 6, **caractérisé en ce qu'**il est réalisé par un procédé continu, et la réaction du phosgène avec l'amine a lieu dans la phase liquide.

8. Procédé selon l'une quelconque des revendications 1 à 7, **caractérisé en ce que** le rapport molaire entre le phosgène et les groupes amino est de 1,1:1 à 12:1.
